# EUROPEAN PATENT APPLICATION

(11) **EP 4 358 093 A1**
(43) Date of publication of application: **24.04.2024**
(21) Application number: 23204166.5
(22) Date of filing: 17.10.2023
(51) Int. Cl.: G16H 40/63, G16H 50/20

(54) **MEDICAL INFORMATION PROCESSING APPARATUS, MEDICAL INFORMATION PROCESSING METHOD AND MEDICAL INFORMATION PROCESSING PROGRAM**

(30) Priority: 18.10.2022 JP 2022167087; 08.08.2023 JP 2023129386
(71) Applicant: Canon Medical Systems Corporation, Tochigi 324-0036 (JP)
(72) Inventor: NORO, Kazumasa, Otawara-shi, 324-0036 (JP); WARIISHI, Nao, Otawara-shi, 324-0036 (JP); SAITO, Hiroki, Otawara-shi, 324-0036 (JP)
(74) Representative: Marks & Clerk LLP

(57) **Abstract**

According to one embodiment, a medical information processing apparatus (1) comprising processing circuitry (10). The processing circuitry (10) acquires (101) an input signal relating to at least one of a treatment and an examination for a subject, the input signal being selected from speech and an input provided by a device used to monitor or provide treatment the subject. The processing circuitry (10) specifies (102), based on the input signal, a treatment or examination that was executed, and a treatment or examination that is to be next executed. The processing circuitry (10) displays (104) a remaining time relating to the treatment or examination to be next executed, and a kind of the executed treatment or examination.

## Description

### FIELD

Embodiments described herein relate generally to a medical information processing apparatus, a medical information processing method and a medical information processing program.

### BACKGROUND

In an emergency medical treatment, numerical data relating to biological information is displayed as conventional technology. However, in a treatment such as cardiopulmonary resuscitation, for example, there is no method of recognizing an elapsed time for each of events such as cardiopulmonary arrest and adrenalin administration, and there is a need for enabling recognition of an elapsed time corresponding to the state of a patient or the status of a treatment.

### SUMMARY

In relation to foregoing embodiments, the following matters are disclosed as one aspect and selected features of the present invention.

In an embodiment, a medical information processing apparatus comprising processing circuitry configured to: acquire an input signal relating to at least one of a treatment and an examination for a subject, the input signal being selected from speech and an input provided by a device used to monitor or provide treatment the subject; specify, based on the input signal, a treatment or examination that was executed, and a treatment or examination that is to be next executed; and display a remaining time relating to the treatment or examination to be next executed, and a kind of the executed treatment or examination.

In an embodiment, the processing circuitry may be further configured to display a timeline indicating a status of a treatment or an examination, including an execution time point of the executed treatment or examination.

In an embodiment, the processing circuitry may update the remaining time, based on the input signal, in a case where an identical treatment or examination to the treatment or examination, the remaining time of which is displayed, is executed once again.

In an embodiment, the processing circuitry may be further configured to display an elapsed time from an execution time point of the executed treatment or examination.

In an embodiment, the processing circuitry may be capable of executing switching between a first display screen displaying the remaining time relating to the treatment or examination to be next executed, and the kind of the executed treatment or examination, and a second display screen indicating time-series data of a vital sign, and the processing circuitry may switch the first display screen to the second display screen, in a case where a specific input signal is acquired.

In an embodiment, the processing circuitry may be further configured to update a content of the treatment or examination to be next executed, in accordance with a number of times of the executed treatment or examination.

In an embodiment, the input signal may be a speech input, the processing circuitry may display, based on the speech signal, an icon on a timeline, the icon indicating an execution time point of the executed treatment or examination.

In an embodiment, in a case where the treatment or examination to be next executed is executed outside an allowable range based on, as a reference, an assumed time instant of the treatment or examination to be next executed, the processing circuitry may display a message confirming whether or not a process is without a problem.

In an embodiment, the processing circuitry may be further configured to display a flowchart along a guideline relating to the treatment or examination for the subject in parallel.

In an embodiment, in a case where a specific situation occurs in regard to at least either the subject or a situation in a room, the processing circuitry may be further configured to execute switching to a second display screen relating to a work flow different from a first display screen displaying the remaining time relating to the treatment or examination to be next executed, and the kind of the executed treatment or examination.

In an embodiment, the processing circuitry may be further configured to reset a count of the remaining time relating to the treatment or examination, or to update a count display in such a manner as to continue the count of the remaining time, in accordance with a kind of the specific situation, in a case of switching the first display screen to the second display screen.

In an embodiment, the processing circuitry may be further configured to: generate confirmation information for re-acquiring the input signal in a case where the input signal is incapable of being acquired; and output the confirmation information by synthesized voice.

In an embodiment, the input signal may be a speech input, the processing circuitry may be further configured to: speech-recognize the speech input and to obtain a speech recognition result; calculate a difference between a time instant at which the speech recognition result was acquired and an assumed time instant of a treatment or examination to be next executed; generate confirmation information if the difference is within a threshold, the confirmation information indicating information as to whether or not the speech input is designation of the treatment or examination to be next executed; display the confirmation information on a screen.

In an embodiment, the processing circuitry may be further configured to output the confirmation information by synthetic voice.

In an embodiment, the device is selected from an electrocardiograph, an ECMO (Extracorporeal membrane oxygenation) device.

In an embodiment, the treatment or examination is selected from at least one of defibrillation, adrenalin administration, cardiac rhythm check, intubation, asystole, VT with pulsation), VT without pulsation.

In an embodiment, a medical information processing method may comprise acquiring an input signal relating to at least one of a treatment and an examination for a subject; specifying, based on the input signal, a treatment or examination that was executed, and a treatment or examination that is to be next executed; and displaying a remaining time relating to the treatment or examination to be next executed, and a kind of the executed treatment or examination.

In an embodiment, a medical information processing program, causing a computer to perform: acquiring an input signal relating to at least one of a treatment and an examination for a subject; specifying, based on the input signal, a treatment or examination that was executed, and a treatment or examination that is to be next executed; and displaying a remaining time relating to the treatment or examination to be next executed, and a kind of the executed treatment or examination.

### BRIEF DESCRIPTION OF THE DRAWINGS

FIG. 1 is a block diagram illustrating a medical information processing apparatus according to a first embodiment.
FIG. 2 is a flowchart illustrating an operation of the medical information processing apparatus according to the first embodiment.
FIG. 3 is a view illustrating a display example of an emergency mode according to the first embodiment.
FIG. 4 is a view illustrating a first display example of a CPA mode according to the first embodiment.
FIG. 5 is a view illustrating a second display example of the CPA mode according to the first embodiment.
FIG. 6 is a view illustrating a third display example of the CPA mode according to the first embodiment.
FIG. 7 is a view illustrating a fourth display example of the CPA mode according to the first embodiment.
FIG. 8 is a view illustrating a fifth display example of the CPA mode according to the first embodiment.
FIG. 9 is a view illustrating a sixth display example of the CPA mode according to the first embodiment.
FIG. 10 is a view illustrating a display example of auxiliary information in the CPA mode.
FIG. 11 is a view illustrating another example of the auxiliary information in the CPA mode.
FIG. 12 is a view illustrating an example of an emergency mode for displaying a CPA mode period.
FIG. 13 is a view illustrating a display example relating to a treatment or examination after a surgical operation.
FIG. 14 is a view illustrating a display example relating to a contrast radiography protocol.
FIG. 15 is a view illustrating a display example relating to hospitalization management.
FIG. 16 is a block diagram illustrating a medical information processing apparatus according to a second embodiment.

### DETAILED DESCRIPTION

In general, according to one embodiment, a medical information processing apparatus comprising processing circuitry. The processing circuitry acquires an input signal relating to at least one of a treatment and an examination for a subject. The processing circuitry specifies, based on the input signal, a treatment or examination that was executed, and a treatment or examination that is to be next executed. The processing circuitry displays a remaining time relating to the treatment or examination to be next executed, and a kind of the executed treatment or examination.

Hereinafter, a medical information processing apparatus, a medical information processing method and a medical information processing program according to embodiments are described with reference to the accompanying drawings. In the embodiments below, parts denoted by identical reference signs are assumed to perform similar operations, and an overlapping description is omitted unless where necessary. One embodiment will now be described with reference to the accompanying drawings.

### (First Embodiment)

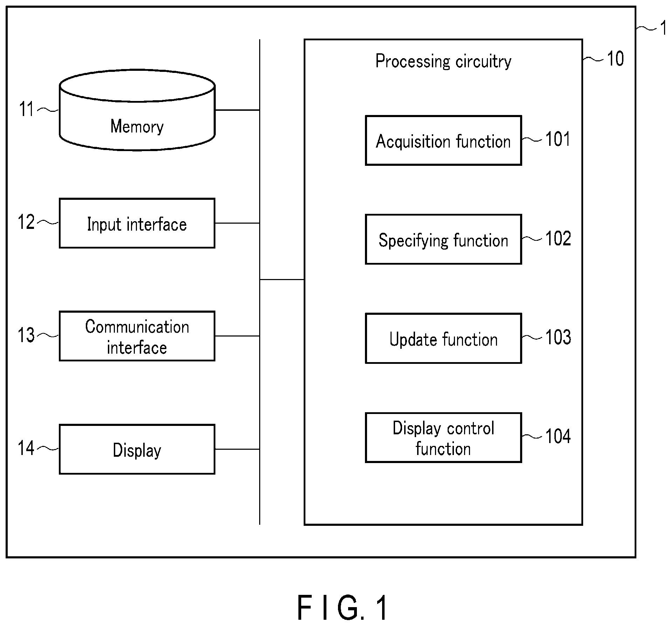

A medical information processing apparatus according to a first embodiment is described with reference to a block diagram of FIG. 1.

A medical information processing apparatus 1 illustrated in FIG. 1 includes processing circuitry 10, a memory 11, an input interface 12, a communication interface 13, and a display 14. The processing circuitry 10, memory 11, input interface 12, communication interface 13 and display 14 are mutually communicably connected, for example, via a bus.

The processing circuitry 10 is a processor functioning as the center of the medical information processing apparatus 1. The processing circuitry 10 is, for example, a processor such as a CPU (Central Processing Unit) or a GPU (Graphics Processing Unit). The processing circuitry 10 includes an acquisition function 101, a specifying function 102, an update function 103, and a display control function 104.

The acquisition function 101 acquires an input signal relating to at least one of a treatment and an examination for a subject. The input signal may be, for example, voice, or a keyboard input.

Based on the input signal, the specifying function 102 specifies a treatment or examination that was executed, and a treatment or examination that is to be next executed.

The update function 103 updates display of a remaining time or an elapsed time on a display screen.

The display control function 104 causes, for example, the display 14 to display a remaining time relating to the treatment or examination to be next executed, and the kind of the executed treatment or examination. In addition, the display control function 104 causes the display 14 to display a timeline indicating the status of a treatment or examination, including an execution time point of the executed treatment or examination.

The memory 11 is a storage device such as a ROM (Read Only Memory), a RAM (Random Access Memory), an HDD (Hard Disk Drive), an SSD (Solid State Drive) or an integrated circuit storage device, which stores various information such as biological information of a patient, a guideline relating to a treatment or examination of a subject, and the like. Besides, the memory 11 may be a CD-ROM drive, a DVD drive, or a drive unit that reads and writes various information from and to a portable storage medium such as a flash memory. Note that the memory 11 may not necessarily be implemented by a single storage device. For example, the memory 11 may be implemented by a plurality of storage devices. In addition, the memory 11 may be provided in some other computer connected to the medical information processing apparatus 1 via a network.

The memory 11 stores a program or the like for executing a process according to the present embodiment. Note that the program may be prestored, for example, in the memory 11. Besides, for example, the program may be stored in a non-transitory storage medium and distributed, and may be read out from the non-transitory storage medium and installed in the memory 11.

The input interface 12 accepts various input operations from a user, converts an accepted input operation into an electric signal, and outputs the electric signal to the processing circuitry 10. The input interface 12 according to the present embodiment is connected to, for example, an input device such as a mouse, a keyboard, a trackball, a switch, a button, a joystick, a touch pad, and a touch panel to which an instruction is input by a touch on an operation surface thereof, or the like. Besides, the input device connected to the input interface 12 may be an input device provided on some other computer that is connected via a network or the like.

The communication interface 13 executes data communication between the medical information processing apparatus 1 and the outside. Although not illustrated, for example, the communication interface 13 executes data communication with a hospital information system, a radiation department information system, or the like. The communication interface 13 executes communication supporting a preset known standard, for example, a standard such as HL7 (Health Level 7) or DICOM (Digital Imaging and Communications in Medicine).

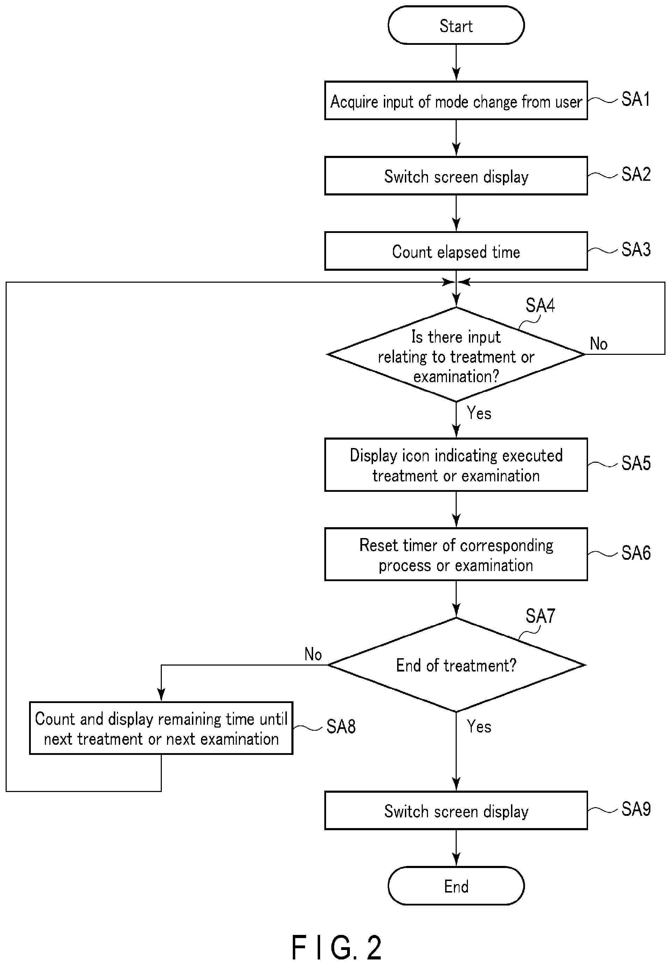

Next, referring to a flowchart of FIG. 2, a description is given of an operation of the medical information processing apparatus 1 according to the first embodiment. Hereinafter, a process in a cardiac arrest mode (hereinafter referred to as "CPA mode") is assumed, and it is assumed that a display screen of an emergency mode is set in advance. The display screen of the emergency mode displays, for example, time-series data of a vital sign.

In step SA1, the processing circuitry 10 acquires, by the acquisition function 101, an input of a mode change from the user. The input of the mode change is accepted, for example, in a case where the user utters a speech "cardiac arrest mode" or "CPA (Cardiopulmonary arrest) mode". Note that aside from the input from the user, a signal from some other device may be used as a trigger of the change. Specifically, for example, in a case where a heart rate measured by an electrocardiograph becomes zero, a trigger signal for a transition to the CPA mode may be transmitted, and, by the processing circuitry 10 receiving the trigger signal by the acquisition function 101, the input of the mode change may be determined, and the emergency mode may transition to the CPA mode.

In step SA2, by the display control function 104, the processing circuitry 10 changes the screen display from the emergency mode to the CPA mode, and displays, on the display screen of the CPA mode, a timer that counts up an elapsed time from the mode change or various events, and a timer that counts down a remaining time until various events. The event refers to a treatment or examination for a patient.

In step SA3, by the display control function 104, the processing circuitry 10 counts up an elapsed time by the timer in accordance with the kind of event, or counts down a remaining time by the timer.

In step SA4, by the specifying function 102, the processing circuitry 10 specifies whether an input relating to a treatment or examination for the patient is present, based on the input signal from the user. For example, in a case where a speech relating to a specific treatment or examination was uttered by a health care worker, it is determined that an input was made. In a case where an input is present, the process advances to step SAS. In a case where an input is absent, the process stands by until an input is made. Like step SA1, based on the presence/absence of a signal from some other device, it may be specified whether an input relating to a process or examination for the patient is present. For example, in a situation in which an ECMO (Extracorporeal membrane oxygenation) is attached to the patient, an operation signal of the ECMO is transmitted to the medical information processing apparatus 1. By the specifying function 102, the processing circuitry 10 may specify, based on the operation signal, that ECMO treatment for the patient was started, and the input of the treatment for the patient was made.

In step SA5, by the display control function 104, the processing circuitry 10 displays an icon indicating an executed treatment or examination on the display screen.

In step SA6, by the display control function 104, the processing circuitry 10 resets the timer of the corresponding process or examination, and resumes the count. Note that in a case where the same process or examination as in the timer displaying the remaining time until the next treatment or examination was executed once again, the processing circuitry 10 updates, by the display control function 104, the remaining time of the timer.

In step SA7, by the specifying function 102, the processing circuitry 10 specifies whether the treatment for the patient ended. For example, in a case where a specific input signal was acquired from a health care worker, it is determined that the treatment for the patient ended. Specifically, in a case where the heartbeat was resumed, the health care worker utters "heartbeat resumed (Shimpaku Saikai)", and thereby it is determined that the treatment in regard to the cardiac arrest treatment for the patient was completed. In a case where the event ended, the process advances to step SA9. In a case where the event does not end, the process advances to step SA8.

In step SA8, by the specifying function 102, the processing circuitry 10 specifies, based on the input signal, the executed treatment or examination, and the treatment or examination to be next executed. By the display control function 104, the processing circuitry 10 executes display such that the remaining time until the next treatment or examination is counted by the timer.

In step SA9, by the display control function 104, the processing circuitry 10 changes the screen display from the CPA mode to the emergency mode.

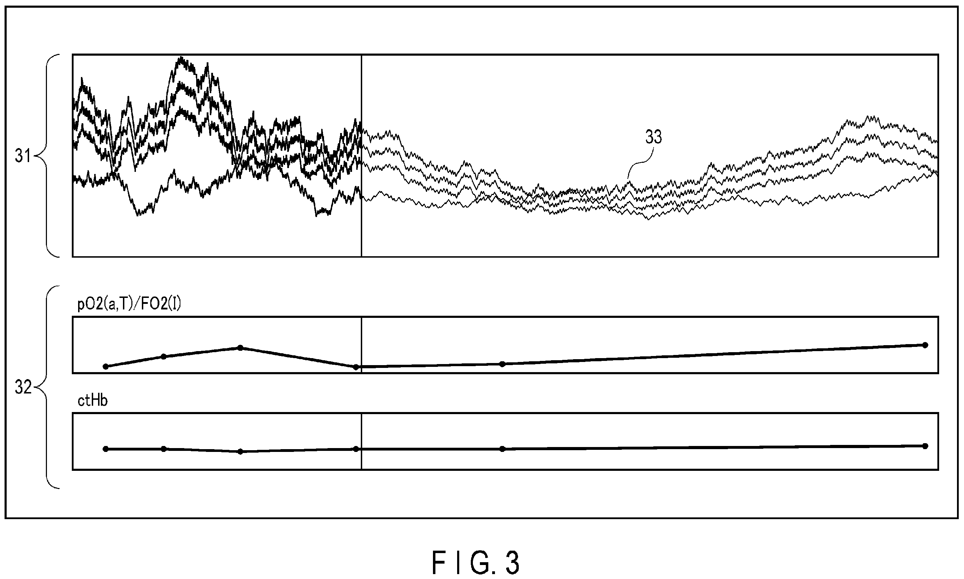

Next, a display example of the emergency mode according to the first embodiment is described with reference to FIG. 3.

FIG. 3 illustrates an example of the display screen, and illustrates a display example of a first display area 31 and a second display area 32. The first display area 31 displays time-series data 33 of vital signs (blood pressure, pulsation, and the like), and the second display area 32 displays time-series data of specific biological information.

The vital data and biological information have the latest measurement values at the right end of the display area, and the time-series data is updated from the right to the left. In the example of FIG. 3, the second display area 32 displays biological information of "pO₂(a, T)/FO₂" and "cTHb" .

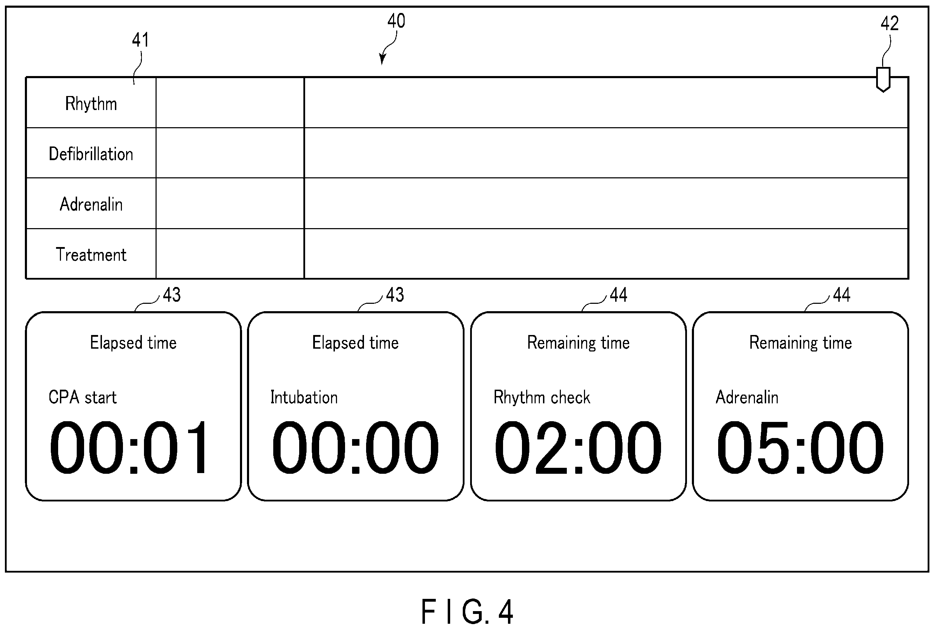

Next, a first display example of the CPA mode according to the first embodiment is described with reference to FIG. 4.

On a display screen 40 of the CPA mode, an upper part displays a timeline, and a lower part displays a plurality of timers.

In the timeline, an icon 42, which indicates the start of the CPA mode, is displayed. Like FIG. 3, a right end of the display area indicates a latest measurement value, and time-series data is updated from the right toward the left. Each time the data is updated, the icon moves from the right to the left. In the timeline, an icon display area of the timeline is displayed in regard to each of events 41. In the example of FIG. 4, four events 41, namely "rhythm", "defibrillation", "adrenalin" and "treatment", are displayed.

The timer 43 counts up an elapsed time from the start of treatment or an elapsed time from an execution time point of an event. The timer 44 counts down a remaining time from the execution of an event. In the example of FIG. 4, the elapsed time from the start of CPA, and the elapsed time from an event "intubation" are counted up by the timers 43, respectively, and the remaining times until the execution of the next events relating to "rhythm check" and "adrenalin administration" are counted down by the timers 44, respectively. In addition, an event 41 of the timeline and a timer corresponding to this event 41 may be correlated by the same color, and different colors may be used for the respective events 41.

Note that although the example is illustrated in which each of the timers 43 and timers 44 is displayed in a card format in which information indicating the distinction between an elapsed time and a remaining time, the kind of event 41, and time information are combined as one set, it is possible to also display the number of times of execution of the event 41 and the biological information of the subject relating to the event. Aside from the card format, the information may be displayed in a table format. In short, any display mode may be used if it is understandable which timer relates to which event.

In addition, the arrangement of the timeline and timers 43 and 44 is not limited to the example of FIG. 4, and the timers 43 and 44, for example, may be arranged vertically.

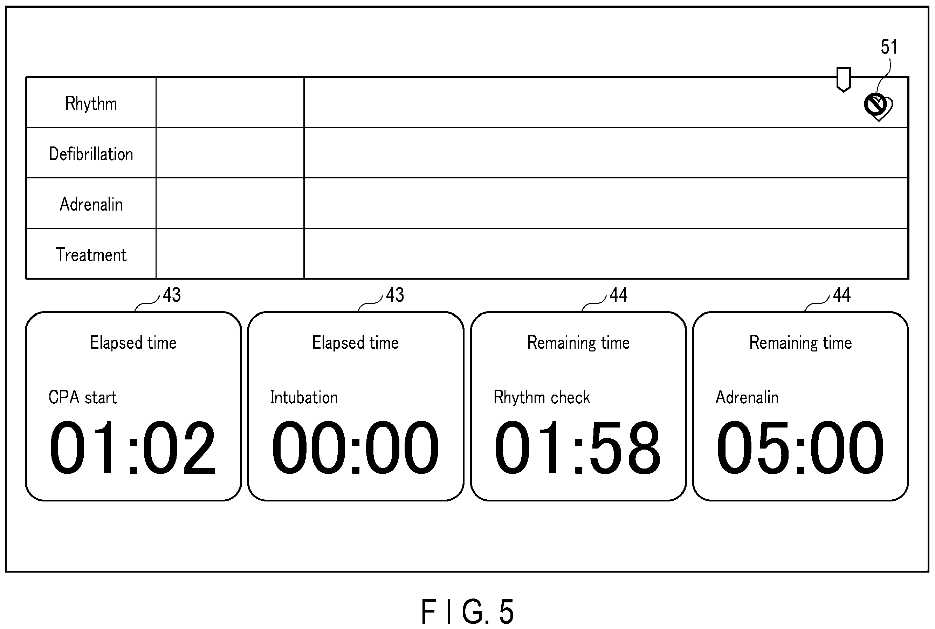

Next, a second display example of the CPA mode according to the first embodiment is described with reference to FIG. 5.

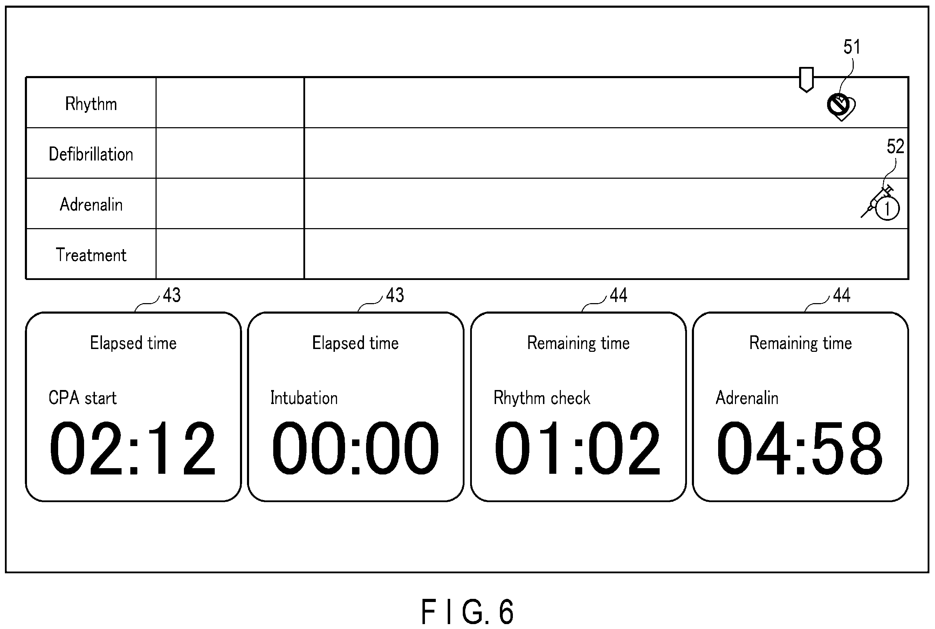

In the example of FIG. 5, in a case where speeches relating to a rhythm check, such as "Rizumu (rhythm)", "Sinseishi (asystole)", "Eisisu (asystole)", "VT", "Myakuari VT (VT with pulsation)", and "Myakunashi VT (VT without pulsation)", were uttered by a health care worker, the processing circuitry 10 specifies, by the specifying function 102, an executed event from the speeches. Here, "Rizumu", "Sinseishi", "Eisis", "Myakuari VT", and "Myakunashi VT" are Japanese utterances. a speech relating to a rhythm check is specified, and the processing circuitry 10, by the display control function 104, displays an icon 51 indicating that the rhythm check was executed, on the timeline of the "rhythm". Note that the shape of the icon illustrated in FIG. 6 onwards is not limited to the example of the icon 51, and may be any shape that is distinguishable from other events.

In addition, in a case where the rhythm check was executed, the processing circuitry 10 resets the timer 44 relating to the rhythm check by the display control function 104, and executes update such that count-down from a set value to the next rhythm check is started. Here, the set value of the timer 44 is two minutes, and in a case where the rhythm check was executed by the above-described speech, the timer 44 executes count-down once again from two minutes.

Next, a third display example of the CPA mode according to the first embodiment is described with reference to FIG. 6.

In the example of FIG. 6, in a case where speeches relating to adrenalin administration, such as "adrenalin", "ADE", "Epinephrine", "Epi", and "Bosmin", were uttered by a health care worker, the processing circuitry 10 specifies, by the specifying function 102, an executed event from the speeches. Here, a speech relating to adrenalin administration is specified, and the processing circuitry 10, by the display control function 104, displays an icon 52 indicating that the adrenalin administration was executed, on the timeline of the "adrenalin". The icon 52 is displayed together with the number of times of administration ("1" in the example of FIG. 6).

In addition, in a case where the adrenalin administration was executed, the processing circuitry 10 resets the timer 44 relating to the adrenalin administration by the display control function 104, and executes update such that count-down from a set value to the next adrenalin administration is started.

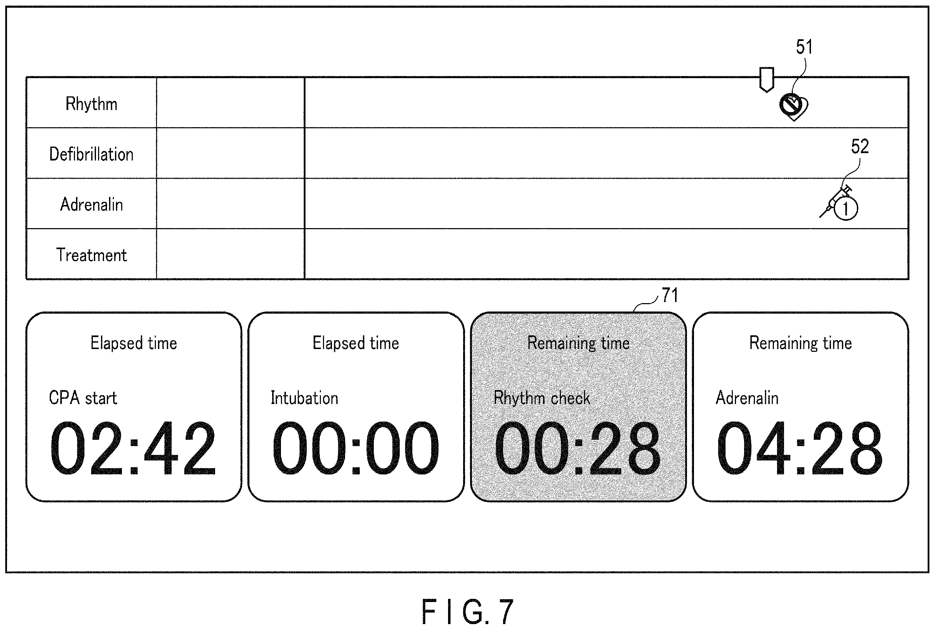

Next, a fourth display example of the CPA mode according to the first embodiment is described with reference to FIG. 7.

FIG. 7 illustrates an example in which a timer 71 relating to a rhythm check is displayed with emphasis, since the remaining time until the execution of the next rhythm check, which is illustrated in FIG. 5, becomes a predetermined time or less. Specifically, if the predetermined time is set at 30 seconds, since the remaining time until the execution of the next rhythm check is 30 seconds or less, the processing circuitry 10 highlight-displays, by the display control function 104, the timer 71 relating to the rhythm check. Note that the entirety of the timer 71 may be highlight-displayed, or a character string of time information may be displayed in red, or a health care worker may be notified of, for instance, "the remaining time until the next rhythm check is 30 seconds", by reading voice. Moreover, in a case where the timer comes to zero, i.e., in a case where the start time of execution of an event has come, reading voice, such as "rhythm check, timer end", or "time has come, start rhythm check", may be output.

Note that in a case where there are a plurality of timers that come to predetermined times and the timings of reading voice overlap, priority may be placed on the timer that first outputs reading voice, and, after the reading ends, reading voice relating to other timers may be output. In this manner, if the health care worker can be notified that a time of the next event is drawing near, any notification mode may be adopted.

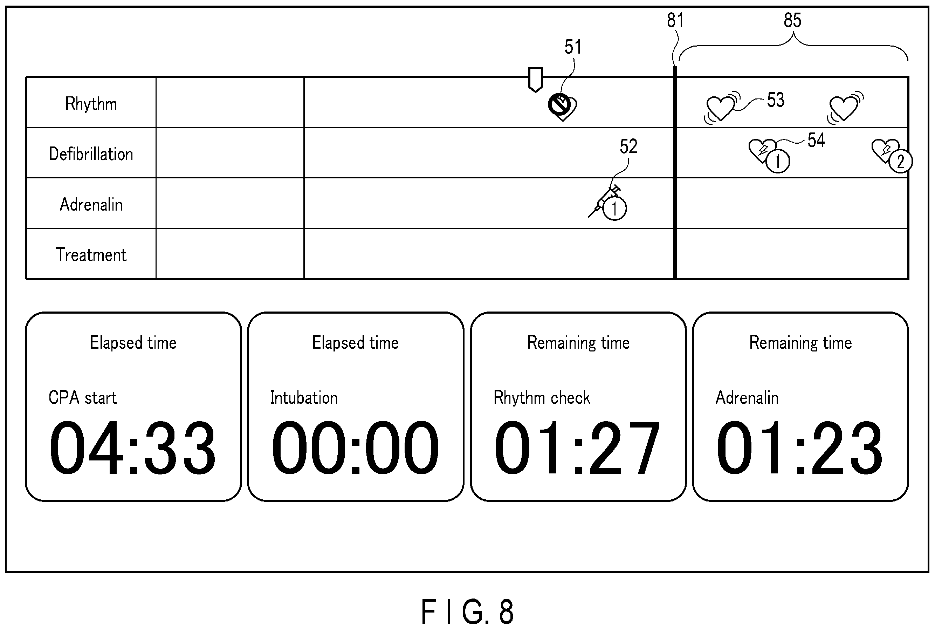

Next, a fifth display example of the CPA mode according to the first embodiment is described with reference to FIG. 8.

In the above-described examples of FIG. 5 to FIG. 7, icons are displayed in a case where events were executed. In the example of FIG. 8, icons are displayed on the timeline in regard to events that are to be executed or may possibly occur.

Specifically, a bar 81 indicating a present time instant is displayed on the timeline. Icons (for example, icons 51 and 52) relating to past events are displayed on the left side of the bar 81, and a plan display area 85 is displayed on the right side of the bar 81, the plan display area 85 displaying icons indicating planned events to be executed. Specifically, the icon 51 of the rhythm check is displayed on the left side of the bar 81, and the rhythm check has already been executed. On the other hand, an icon 53 of the rhythm check and an icon 54 of defibrillation, which indicate events planned to be executed, are displayed on the plan display area 85. By this display, the planned events to be next executed can easily be understood by the icons.

As regards the planned events to be executed, for example, by referring to a guideline stored in the memory 11, in this example, along a guideline for executing cardiopulmonary resuscitation from the cardiac arrest state, the processing circuitry 10 specifies, by the specifying function 102, one or more events to be next executed from an executed event. Thereafter, by the display control function 104, the processing circuitry 10 may display the icons corresponding to the specified events on the timeline in the order of execution or along the intervals of execution.

Note that the processing circuitry 10 may update, by the display control function 104, the planned events to be executed, as appropriate, in accordance with events executed by the health care worker. For example, in the guideline for executing cardiopulmonary resuscitation, the flow may branch, depending on the results of the execution of events. Thus, the processing circuitry 10 may specify, by the specifying function 102, a branch destination flow of the event to be next executed, from the execution result of the event input by the health care worker, and the processing circuitry 10 may display, by the display control function 104, the event relating to the branch destination flow by an icon on the timeline.

Besides, in accordance with the number of times of the executed event, the content of the event to be next executed, and the remaining time set in the timer 44, may be changed. For example, in a case where the content of the event is changed, if the event has been executed a predetermined number of times, the processing circuitry 10 may make a change from a simple examination to a thorough examination by the update function 103. In a case of changing the remaining time of the timer 44, the processing circuitry 10 may increase, by the update function 103, the set value of the remaining time of the timer 44 in such a manner as to increase the time interval until the execution of the next event.

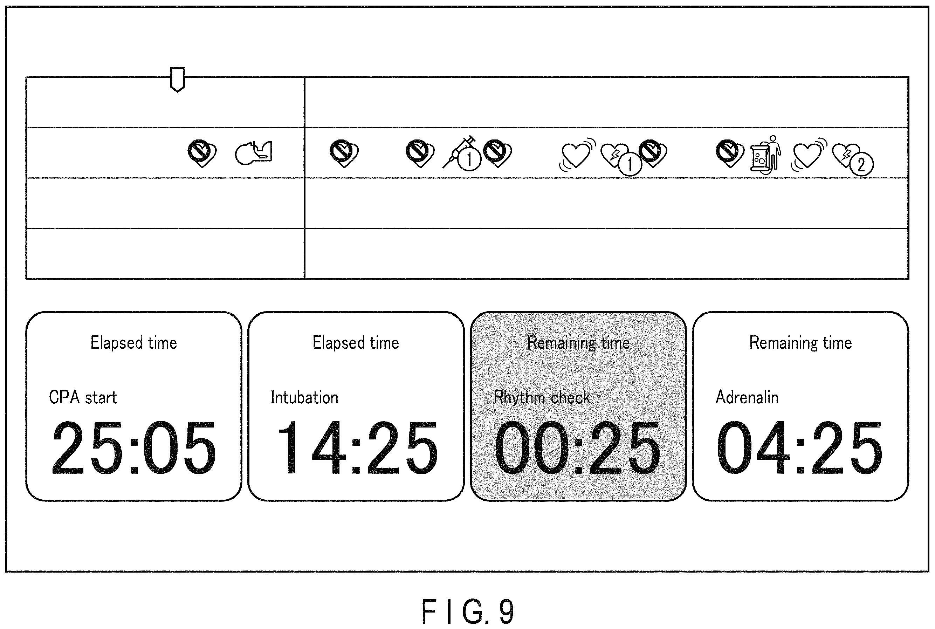

Next, a sixth display example of the CPA mode according to the first embodiment is described with reference to FIG. 9.

Here, an example is illustrated in which a plurality of kinds of icons are displayed in one row, without dividing rows on the timeline, which display icons for individual events. By displaying the icons in one row, it becomes easier to understand the time-series order of execution of events.

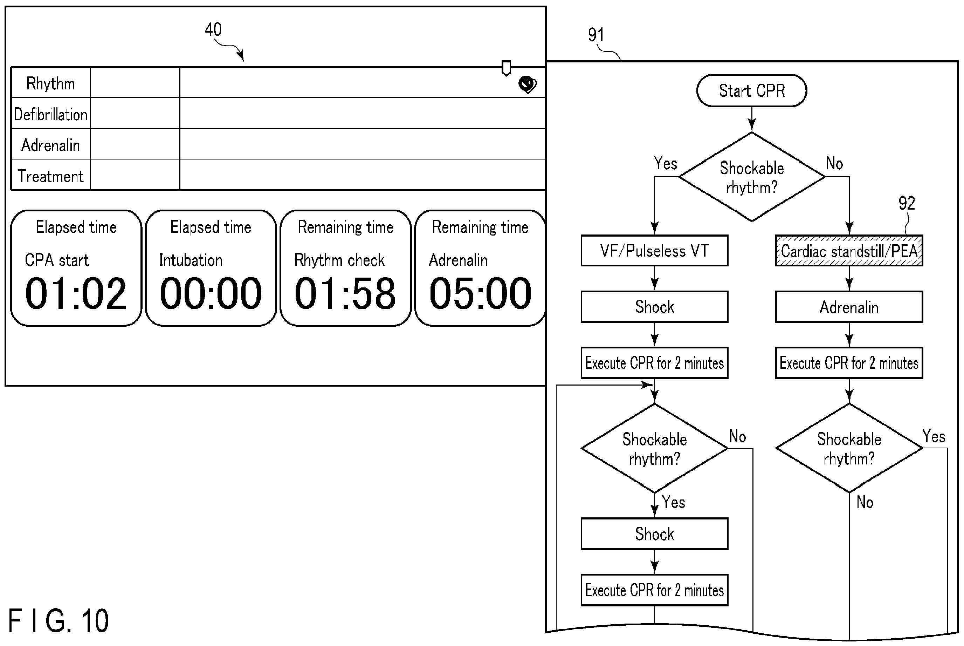

Next, a display example of auxiliary information in the CPA mode is described with reference to FIG. 10.

FIG. 10 illustrates an example in which the display screen 40 of the CPA mode, as illustrated in FIG. 4 to FIG. 9, and a flowchart 91 illustrating a work flow along the guideline, are displayed in parallel. In the example of FIG. 10, the flowchart 91 is, for example, a flowchart of a cardiac arrest algorithm along the guideline stipulated by an academic conference and an association relating to the heart. The health care worker refers to the displayed flowchart 91 while executing a treatment or examination relating to an event, and thereby the confirmation of the work flow becomes easier and the speed-up of the treatment can be supported.

Furthermore, by the display control function 104, the processing circuitry 10 may display with emphasis, on the flowchart 91, an event 92 of the work flow corresponding to the event executed most lately. Thereby, the health care worker can easily understand which event is being executed at present. Aside from the present treatment or examination, the event to be next executed may be displayed with emphasis. Note that, aside from the flowchart format, the guideline or work flow may be displayed in a list format, and any display mode may be adopted if the work flow can be recognized.

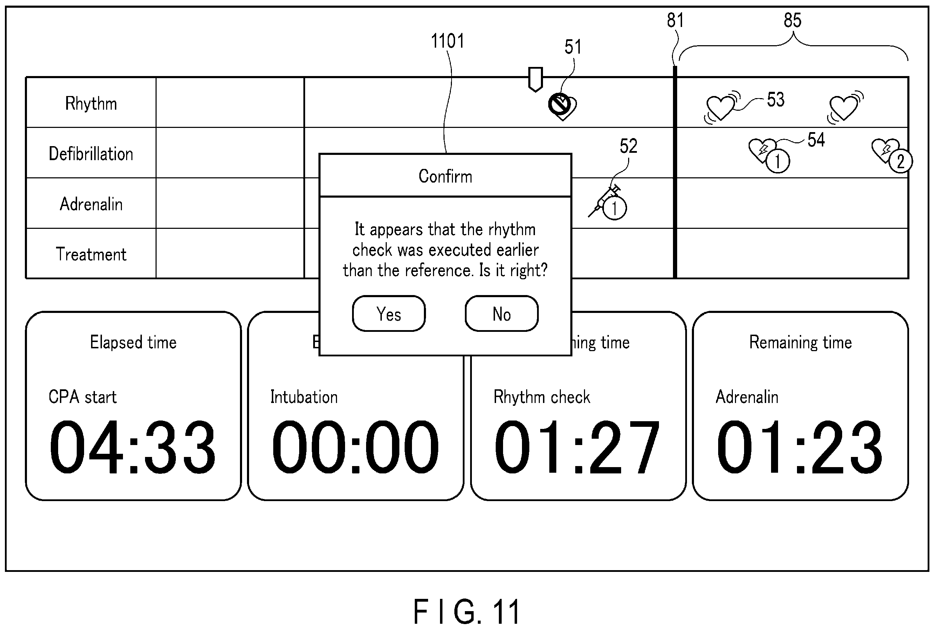

Next, another example of the auxiliary information in the CPA mode is described with reference to FIG. 11.

The remaining time until the next event is counted by the timer, and if the event was executed at a time outside an allowable range based on, as a reference, an assumed time instant of the event to be next executed, that is, if the event was executed earlier than a predetermined time or later than the predetermined timing, a message 1101 is displayed which prompts confirmation as to whether there is a problem with the timing of the execution.

The example of FIG. 11 assumes a case where the event of the rhythm check was executed before a predetermined time has not yet passed by the timer that counts down the remaining time. In this case, by the display control function 104, the processing circuitry 10 displays on the screen the message 1101 including the content "It appears that the rhythm check was executed earlier than the reference. Is it right?"

Thereby, the health care worker can be prompted to confirm whether the execution of the event is right.

Note that in a case of switching the screen display from the CPA mode to the emergency mode, the period in which the CPA mode was executed may be displayed on the screen of the emergency mode.

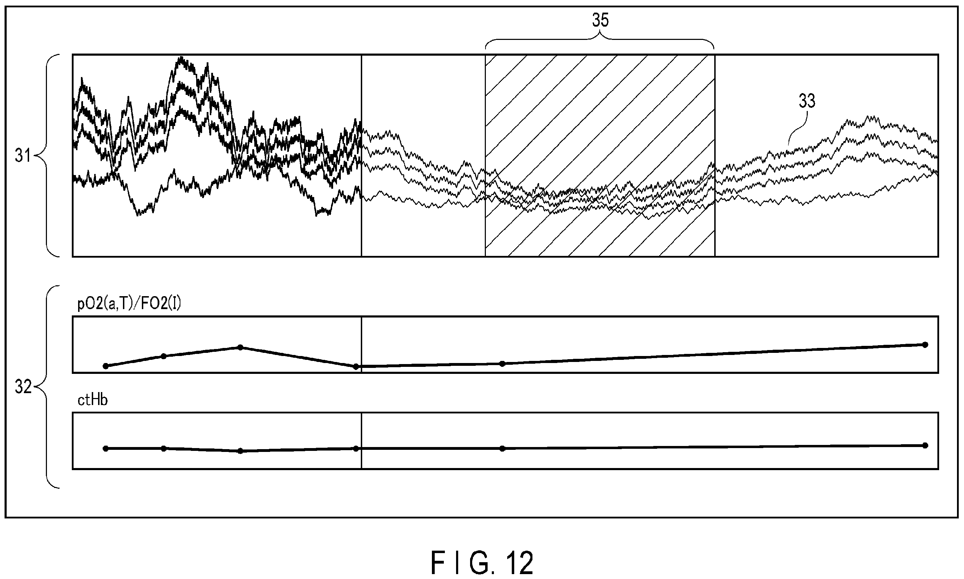

A display example of the emergency mode, which displays the execution period of the CPA mode, is described with reference to FIG. 12.

FIG. 12 illustrates an example in which an execution period 35 of the CPA mode is displayed by hatching in the first display area 31 that displays vital signs, like FIG. 3. Needless to say, the display may be effected in such a mode that the execution period 35 of the CPA mode and another period can be distinguished, for instance, by changing the background color of the execution period 35, or by flickering. Thereby, simply by viewing the display screen of the emergency mode, it becomes possible to easily understand in which period the CPA occurred.

The above-described examples illustrate display examples relating to cardiac arrest (CPA). Aside from this, the medical information processing apparatus according to the present embodiment can similarly support, as well as the medical practice in the emergency, other medical practice such as event management of medication, a vital check and the like in an intensive care unit (ICU).

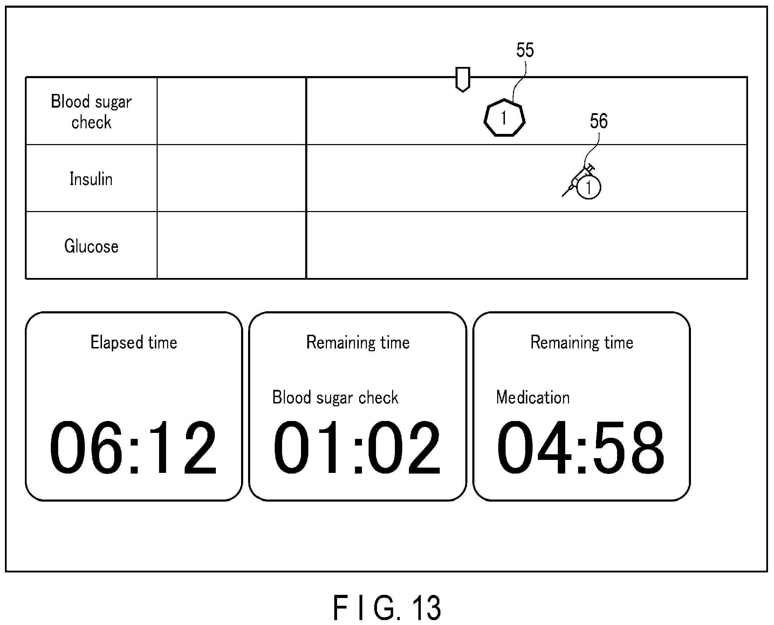

Referring to FIG. 13, a description is given of a display example in a case of using the process of the medical information processing apparatus 1 for a treatment or examination after a surgical operation.

FIG. 13 illustrates a display screen relating to monitoring of a blood sugar level after a surgical operation. An upper part of the display screen displays a timeline of events relating to a blood sugar level check, insulin administration, and glucose administration. A lower part of the display screen displays, as timers, an elapsed time after a surgical operation, a remaining time until a blood sugar level check, and a remaining time until administration (for example, insulin). Here, an icon 55 of a blood sugar level check and an icon 56 of insulin administration are displayed, and it is understood that each of the blood sugar level check and the insulin administration was executed once. In this manner, the medical information processing apparatus 1 according to the present embodiment can be utilized for follow-up after a surgical operation.

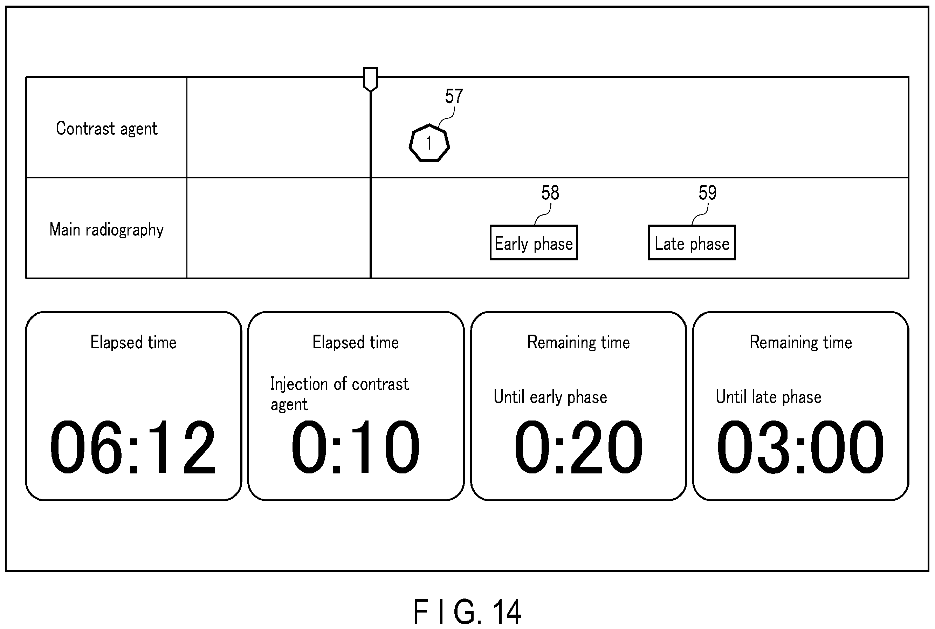

Next, referring to FIG. 14, a description is given of an example in which the process of the medical information processing apparatus 1 is used for a contrast radiography protocol.

FIG. 14 illustrates an example in which timings from the injection of a contrast agent to main radiography are displayed by a timeline and timers. An upper part of the display screen displays a timeline of events relating to a contract medium and main radiography. A lower part of the display screen displays an elapsed time from the start of an examination, an elapsed time from the injection of the contact medium, a remaining time until an early phase, and a remaining time until a late phase. In the example of FIG. 14, an icon 57 indicating the injection of the contrast agent is displayed on the timeline. In addition, an icon 58 indicating a timing of the early phase, and an icon 59 indicating a timing of an equilibrium phase are displayed on the timeline. In this manner, the timer indicating the remaining time for main radiography from the time instant of the injection of the contrast agent may be displayed together with the timeline indicating the progress of the radiography protocol (the list of events such as contrast agent injection and radiography).

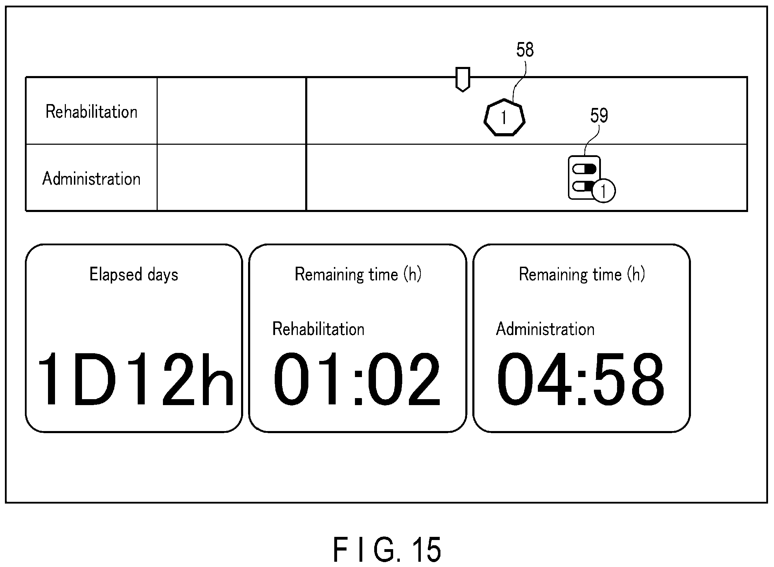

Next, referring to FIG. 15, a description is given of an example in which the process of the medical information processing apparatus 1 is used for hospitalization management.

FIG. 15 illustrates an example in which timings of various events are displayed by a timeline and timers, by taking as an example an inpatient that requires rehabilitation due to a surgical operation of bone fracture, ligament injury or the like. An upper part of the display screen displays a timeline of events relating to execution of rehabilitation, and administration (or medication). A lower part of the display screen displays an elapsed time from hospitalization, a remaining time until the start of rehabilitation, and a remaining time until medication. In the example of FIG. 14, an icon 58 indicating that rehabilitation was executed, and an icon 59 indicating that medication was taken, are displayed on the timeline. Moreover, a timeline and a timer of other events such as a vital check may similarly be displayed. Thereby, the timing of rehabilitation, and medication, which are to be managed by the patient himself/herself, can be notified. Thus, forgetting to take medication, or the like, can be prevented, and an event that needs to be periodically executed can be managed.

Note that, aside from an inpatient, even in a case of remote medical care such as home medical care, the event management of the medical information processing apparatus 1, which is represented by the above-described rehabilitation and administration management, can similarly be applicable.

In addition, in the above-described example, the switching between the emergency mode and the CPA mode was described by way of example, but the work flow may be switched in accordance with a specific situation such as a rare case. Specifically, the work flow may be switched in a case where a treatment method changes due to a specific situation or some change of a state, such as in a case where a patient fell from the bed, the condition of a patient suddenly changed, or cardiac infarction, which was treated, turned out to be actually brain infarction.

In a case where a specific situation occurs in regard to at least either a patient that is a subject or a situation of a room such as an operation room where the patient is present, the processing circuitry may switch, by the display control function 104, the display screen of the timeline of the treatment or examination relating to the event of a work flow to a display screen of a timeline of a treatment or examination relating to the event of a different work flow.

As regards the switching method of the display screen, for example, the work flow may be switched in response to voice, or a change of the state of the patient may be detected. As regards the method of detecting the state of the patient, for example, a camera capable of photographing the scene in the room may be installed, and the state of the patient may be obtained from the acquired camera image. For example, the state of the patient may be obtained by detecting a change, based on the state of the patient during the operation, a change of a surgical instrument, or the like. Besides, the work flow may be switched by detecting a change (for example, the heart rate is zero) of the situation or the state of the patient, based on vital signs measured by a living body monitor, or the like. Specifically, by the display control function 104, the processing circuitry 10 may execute switching to the CPA mode, if cardiac arrest is detected by the vital sign of the heartbeat in a case of treating brain infarction.

In addition, by the display control function 104, the processing circuitry 10 may input and display an event, such as a change of an intravenous drip, which can automatically be detected by the system by the above-described camera image or living body monitor, in a mode distinguishable from a user input by a health care worker, such as a gray display mode. In this case, by the acquisition function 101, the processing circuitry 10 may accept a confirmation operation such as a touch on a predetermined area of the display, or depression of a button, by the health care worker via the input interface 12, and thereby the automatically detected event may be determined. Besides, input person information indicating who made an input may be included in regard to all event inputs.

Note that in a case of switching the display screen in accordance with the switching of the work flow, the processing circuitry 10 may reset, by the update function 103, the count of the timer, or may update the count display so as to continue the count of the remaining time, in accordance with the kind of the specific situation. For example, the timer relating to the timing of an electrocardiogram or a vital check has no restrictions due to the number of times of measurement or the timing of measurement, and thus may be reset in accordance with the switching of the work flow. Meanwhile, since the administration of adrenalin has restrictions in regard to administration, such as a predetermined number of times, or a predetermined time interval, it is better to continuously count, even if the working flow is switched. Thus, even if the work flow is switched, the remaining time may be carried over, and the count may be continued.

Note that a table, in which the kind of a specific situation and information indicating whether to reset or continue the timer are correlated, may be stored in the memory 11 or the like in regard to each of work flows, and the processing circuitry 10 may determine, by the update function 103, the reset or the continuation of the timer relating to the above-described event, by referring to the table in accordance with the switching of the work flow. In this manner, the time management suited to the situation can be supported by setting the count of the timer relating to the event of the next executed treatment or examination in accordance with the kind of the specific situation.

According to the above-described first embodiment, an executed treatment or examination and a next executed treatment or examination are specified based on an input signal relating to at least one of a treatment and an examination for a subject, which was executed by a health care worker. A remaining time relating to one or more treatments or examinations to be next executed is displayed, and the kind of the executed treatment or examination is displayed.

Thereby, it is possible to support time management relating to each event of a work flow, as to which of events, such as treatments or examinations, is to be preferably executed at which timing.

### (Second Embodiment)

A second embodiment assumes a process in a case where an event that was input by voice from a health care worker could not be specified.

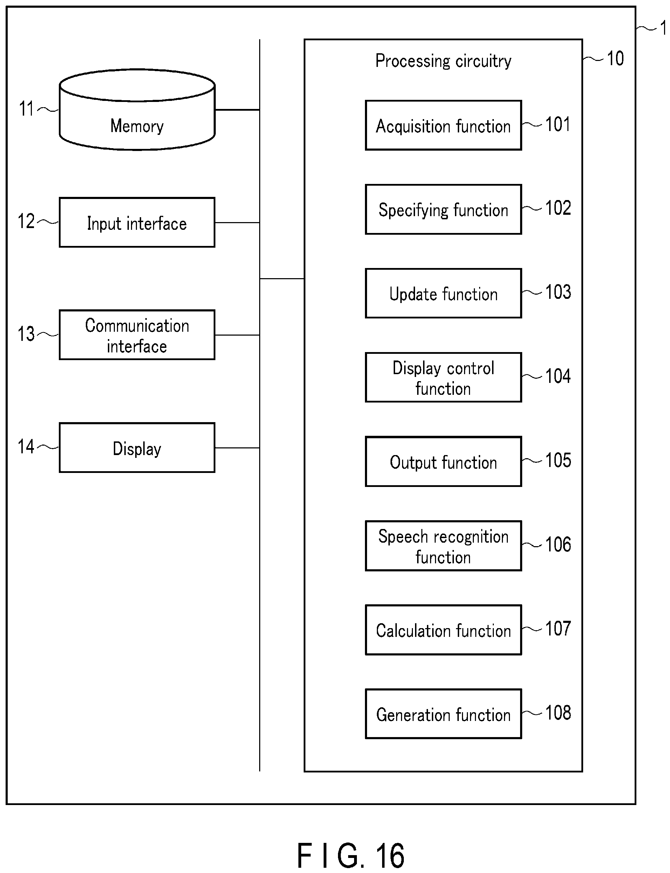

A medical information processing apparatus 1 according to the second embodiment is described with reference to a block diagram of FIG. 16.

The medical information processing apparatus 1 according to the second embodiment includes processing circuitry 10, a memory 11, an input interface 12, a communication interface 13, and a display 14. The processing circuitry 10 includes an acquisition function 101, a specifying function 102, an update function 103, a display control function 104, an output function 105, a speech recognition function 106, a calculation function 107, and a generation function 108.

In a case where a speech input was received from the health care worker but the speech input could not be acquired, such as in a case where the speech input cannot be recognized as a speech due to ambient noise, the processing circuitry 10 generates, by the specifying function 102, confirmation information for acquiring a speech input once again. The confirmation information may be, for example, a message prompting the health care worker to utter a speech, such as "Speech could be heard. Please speak once again." Alternatively, the confirmation information may be a message prompting the health care worker to make an input via other input means, such as "Speech could be heard. Please select an event."

By the output function 105, the processing circuitry 10 outputs the confirmation information generated by the specifying function 102, by synthesized voice, to the outside via an output device (not illustrated) such as speaker.

By the speech recognition function 106, the processing circuitry 10 speech-recognizes a speech input of the health care worker, and obtains a speech recognition result.

By the calculation function 107, the processing circuitry 10 calculates a difference between a time instant at which the speech recognition result was acquired by the speech recognition function 106 and an assumed time instant of an event to be next executed.

By the generation function 108, the processing circuitry 10 generates, if the difference is within a threshold, confirmation information as to whether the speech input is the event to be next executed. The confirmation information may be a message for confirming a specified event, such as "Medication, is it right?".

The display control function 104 displays the confirmation information on the screen. In addition, by the output function 105, the processing circuitry 10 may output synthesized voice relating to the confirmation information to the outside via the output device.

Note that, instead of displaying the confirmation information, an icon of the event may be displayed on the screen in gray, since the event acquired as the speech recognition result is highly possibly the event of the assumed time instance. In this case, by the acquisition function 101, the processing circuitry 10 may accept a confirmation operation such as a touch on a predetermined area of the display, or depression of a button, by the health care worker via the input interface 12, and thereby the automatically detected event may be determined. Alternatively, in a case where the remaining time of the timer becomes zero and there is no input even after a predetermined time has passed since the timing at which the event is to be input, the processing circuitry 10 prompts the input of, or the re-acquisition of, the event by the display control function 104.

According to the above-described second embodiment, in a case where the input of an event by a user's speech could not be specified, the generation function gives a feedback to the user and prompts the user to make a correct input. Alternatively, by referring to the difference between a time instant of the acquisition of the speech recognition result and the next event near the time instant, if the difference is within the threshold, which of events is to be input is specified and asked once again. Thereby, an exact event input and timer count can be executed, and, like the first embodiment, time management can be supported.

Note that the term "processor" used in the above description means, for example, a CPU (Central Processing Unit), a GPU (Graphics Processing Unit), or circuitry such as an application specific integrated circuit (ASIC) or a programmable logic device (e.g., simple programmable logic device (SPLD), a complex programmable logic device (CPLD) or a field programmable gate array (FPGA)). If the processor is, for example, a CPU, the processor implements the functions by reading and executing the program stored in the storage circuitry. On the other hand, if the processor is, for example, an ASIC, the functions are directly incorporated in the circuitry of the processor as logic circuitry, instead of the programs being stored in the storage circuitry. Note that, aside from the case where each of the processors of the embodiments is constructed as single circuitry for each processor, the processors may be constructed as a single processor by combining a plurality of independent circuits and thereby the functions may be implemented. Furthermore, a plurality of structural elements in the drawings may be integrated into a single processor, and the functions thereof may be implemented.

In addition, each of the functions according to the embodiments can also be implemented by installing the programs that execute the above-described process into a computer such as a workstation, and loading the programs on the memory. At this time, the programs that can cause the computer to execute the above-described methods can be distributed by being stored in storage media such as a magnetic disk (hard disk or the like), an optical disc (CD-ROM, DVD or the like), a semiconductor memory or the like.

According to at least one of the above-described embodiments, time management can be supported.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical information processing apparatus (1) comprising processing circuitry (10) configured to:
acquire (101) an input signal relating to at least one of a treatment and an examination for a subject, the input signal being selected from speech and an input provided by a device used to monitor or provide treatment the subject;
specify (102), based on the input signal, a treatment or examination that was executed, and a treatment or examination that is to be next executed; and
display (104) a remaining time relating to the treatment or examination to be next executed, and a kind of the executed treatment or examination.

2. The medical information processing apparatus (1) according to Claim 1, wherein the processing circuitry (10) is further configured to display a timeline indicating a status of a treatment or an examination, including an execution time point of the executed treatment or examination.

3. The medical information processing apparatus (1) according to Claim 1, wherein the processing circuitry (10) is configured to update the remaining time, based on the input signal, in a case where an identical treatment or examination to the treatment or examination, the remaining time of which is displayed, is executed once again.

4. The medical information processing apparatus (1) according to Claim 1, wherein the processing circuitry (10) is further configured to display an elapsed time from an execution time point of the executed treatment or examination.

5. The medical information processing apparatus (1) according to Claim 1, wherein
the processing circuitry (10) is capable of executing switching between a first display screen displaying the remaining time relating to the treatment or examination to be next executed, and the kind of the executed treatment or examination, and a second display screen indicating time-series data of a vital sign, and
the processing circuitry (10) switches the first display screen to the second display screen, in a case where a specific input signal is acquired.

6. The medical information processing apparatus (1) according to Claim 1, wherein the processing circuitry (10) is further configured to update a content of the treatment or examination to be next executed, in accordance with a number of times of the executed treatment or examination.

7. The medical information processing apparatus (1) according to Claim 1, wherein
the input signal is a speech input, and
the processing circuitry (10) is configured to display, based on the speech signal, an icon on a timeline, the icon indicating an execution time point of the executed treatment or examination.

8. The medical information processing apparatus (1) according to Claim 1, wherein in a case where the treatment or examination to be next executed is executed outside an allowable range based on, as a reference, an assumed time instant of the treatment or examination to be next executed, the processing circuitry (10) displays a message confirming whether or not a process is without a problem.

9. The medical information processing apparatus (1) according to Claim 1, wherein the processing circuitry (10) is further configured to display a flowchart along a guideline relating to the treatment or examination for the subject in parallel.

10. The medical information processing apparatus (1) according to Claim 1, wherein in a case where a specific situation occurs in regard to at least either the subject or a situation in a room, the processing circuitry (10) is further configured to execute switching to a second display screen relating to a work flow different from a first display screen displaying the remaining time relating to the treatment or examination to be next executed, and the kind of the executed treatment or examination.

11. The medical information processing apparatus (1) according to Claim 10, wherein the processing circuitry (10) is further configured to reset a count of the remaining time relating to the treatment or examination, or to update a count display in such a manner as to continue the count of the remaining time, in accordance with a kind of the specific situation, in a case of switching the first display screen to the second display screen.

12. The medical information processing apparatus (1) according to Claim 1, wherein the processing circuitry (10) is further configured to:
generate confirmation information for re-acquiring the input signal in a case where the input signal is incapable of being acquired; and
output the confirmation information by synthesized voice.

13. The medical information processing apparatus (1) according to Claim 1, wherein
the input signal is a speech input,
the processing circuitry (10) is further configured to:
speech-recognize (106) the speech input and to obtain a speech recognition result;
calculate (107) a difference between a time instant at which the speech recognition result was acquired and an assumed time instant of a treatment or examination to be next executed;
generate (108) confirmation information if the difference is within a threshold, the confirmation information indicating information as to whether or not the speech input is designation of the treatment or examination to be next executed; and
display the confirmation information on a screen.

14. The medical information processing apparatus (1) according to Claim 13, wherein the processing circuitry (10) is further configured to output (105) the confirmation information by synthetic voice.

15. A medical information processing method comprising:
acquiring an input signal relating to at least one of a treatment and an examination for a subject, the input signal being selected from speech and an input provided by a device used to monitor or provide treatment the subject;
specifying, based on the input signal, a treatment or examination that was executed, and a treatment or examination that is to be next executed; and
displaying a remaining time relating to the treatment or examination to be next executed, and a kind of the executed treatment or examination.

16. A medical information processing program, causing a computer to perform:
acquiring an input signal relating to at least one of a treatment and an examination for a subject;
specifying, based on the input signal, a treatment or examination that was executed, and a treatment or examination that is to be next executed; and
displaying a remaining time relating to the treatment or examination to be next executed, and a kind of the executed treatment or examination.
